(19) Europäisches Patentamt
European Patent Office
Office européen des brevets

(11) Veröffentlichungsnummer: **0 000 879**
**B1**

(12) **EUROPÄISCHE PATENTSCHRIFT**

(21) Anmeldenummer: **78100570.7**

(22) Anmeldetag: **02.08.78**

(51) Int. Cl.³: **C 07 C 69/96,**
C 07 C 68/06,
B 01 J 31/12, //
C 08 G 63/62

(54) **Verfahren zur Herstellung aromatischer Kohlensäureester**

(30) Priorität: **10.08.77 DE 2736062**

(43) Veröffentlichungstag der Anmeldung:
**07.03.79 Patentblatt 79/05**

(45) Bekanntmachung des Hinweises auf die Patenterteilung:
**20.08.80 Patentblatt 80/17**

(84) Benannte Vertragsstaaten:
**DE FR GB NL**

(56) Entgegenhaltungen:
**DE - A - 2 528 412**
**DE - A - 2 552 907**
**DE - A - 2 725 255**
**US - A - 3 714 234**

(73) Patentinhaber: **Bayer AG**
**Zentralbereich Patente, Marken und Lizenzen**
**D - 5090 Leverkusen 1, Bayerwerk (DE)**

(72) Erfinder: **Krimm, Heinrich, Dr.**
**Heyenbaumstrasse 65**
**D - 4150 Krefeld (DE)**
**Buysch, Hans-Josef, Dr.**
**Brandenburger Strasse 28**
**D - 4150 Krefeld (DE)**
**Rudolph, Hans, Dr.**
**Haydnstrasse 9**
**D - 4150 Krefeld (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung aromatischer Kohlensäureester

Die Erfindung betrifft ein Verfahren zur Herstellung aromatischer Kohlensäureester aus aliphatischen Kohlensäureestern und Phenolen durch Umesterung in Gegenwart von zinnorganischen Verbindungen.

Die Umesterung aliphatischer Kohlensäureester mit Phenolen in Anwesenheit starker Basen bzw. von Alkaliverbindungen ist nach DBP 971 790, 1 020 184, 1 026 958 und 1 031 512 bekannt. Derart katalysierte Umesterungsverfahren haben den Nachteil, wenig selektiv zu sein, so daß in einer Nebenreaktion erhebliche Mengen an Kohlendioxid freigesetzt werden.

In den DOS 2 528 412 und 2 552 907 sind Umesterungsverfahren zur Herstellung aromatischer Kohlensäureester beschrieben, bei denen als Katalysatoren Lewissäuren, d.h. Übergangsmetallhalogenide oder die entsprechenden Acyloxy-, Alkoxy- oder Aryloxy - Verbindungen verwendet werden. Von den Verbindungen der Elemente Al, Ti, U, V, Zn, Fe und Sn sind nur die des Titans von wirtschaftlichem Interesse, da nur diese hinreichend wirksam und selektiv sind. Diese Katalysatoren auf Titanbasis haben aber den Nachteil, daß sie die Endprodukte stark rotbraun färben. Diese Färbung tritt besonders dann unangenehm in Erscheinung, wenn sich die Endprodukte nicht durch Umkristallisieren oder Destillieren reinigen lassen, wie das z.B. bei Polycarbonaten der Fall ist.

Die Us-Patentschrift 3 714 234 lehrt nun, Carbonsäureester in Anwesenheit bestimmter Zinnverbindungen umzuestern. Diese Zinnverbindungen sind aber Umsetzungsprodukte von organischen oder anorganischen Zinnverbindungen mit Alkalimetall - Alkoxiden oder Phenoxiden. Die Produkte enthalten auf jeden Fall in irgendeiner Form Alkali, sind damit basisch und begünstigen die $CO_2$-Abspaltung in hohem Maße. Es ist Klar, daß diese Katalysatoren für die Umesterung von Kohlensäureestern nicht in Frage kommen.

Es war deshalb die Aufgabe gestellt, Katalysatoren zu finden, die neben einer zumindest den Titankatalysatoren gleichwertigen Umesterungswirksamkeit und Selektivität auch den Vorteil aufweisen, die Endprodukte nicht wesentlich zu verfärben.

Die erfindungsgemäße Lösung der Aufgabe gelang durch die Verwendung bestimmter zinnorganischer Verbindungen als Umesterungskatalysatoren. Gegenstand der Erfindung sind daher Verfahren zur Herstellung von aromatischen Kohlensäureestern durch Umesterung von Dialkylcarbonaten mit Phenolen unter Abspaltung von Alkoholen in Gegenwart von Katalysatoren, die dadurch gekennzeichnet sind, daß als Katalysatoren zinnorganische Verbindungen der allgemeinen Formel (I)

$$(R^1)_{4-x}-Sn-(Y)_x \qquad (I),$$

in der

Y für einen Rest

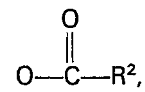

OH oder $OR^2$ steht, wobei $R^2$ einen Alkylrest mit $C_1$—$C_{12}$, einen Arylrest mit $C_6$—$C_{12}$ oder einen Alkylarylrest mit $C_7$—$C_{13}$ bedeutet, und

$R^1$ die Bedeutung von $R^2$ hat, und

x eine ganze Zahl von 1—3 bedeutet,

oder Dialkylzinnoxide mit jeweils 1—12 C-Atomen im Alkylrest oder zinnorganische Verbindungen der allgemeinen Formel (II)

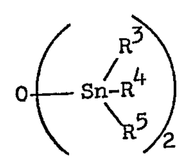

(II),

in der

$R^3$ und $R^4$ gleich oder verschieden, die oben angegebene Bedeutung von $R^2$ haben, und

$R^5$ die Bedeutung von $R^2$ hat oder für einen Rest $OR^6$ steht, in dem $R^6$ die Bedeutung von $R^2$ hat,

werwendet werden.

Die günstige katalytische Wirkung der erfindungsgemäßen Katalysatoren ist überraschend, da man sie nicht als Lewissäuren bezeichnen kann. Ganz im Gegenteil ist festzustellen, daß gerade die zinnorganischen Halogenverbindungen, wie Dibutylzinndichlorid oder Dioctylzinndichlorid, bei denen noch am ehesten eine formale Verwandtschaft zu typischen Lewissäuren wie Aluminiumtrichlorid oder Titantetrachlorid zugerechnet werden könnte, völlig unwirksam sind. Die erfindungsgemäß verwendeten zinnorganischen Verbindungen dürfen daher keine direkte Zinn - Halogen - Verbindung enthalten.

Die Wirksamkeit der erfindungsgemäßen Katalysatoren, gemessen an der Geschwindigkeit der Alkoholabspaltung, unterscheidet sich praktisch nicht von der von Verbindungen des Titans wie z.B. des Tetrabutyltitanats. Doch sind Vorteile darin zu sehen, daß in den beiden Verfahrensprodukten Alkylarylcarbonat und Diarylcarbonat das Verhältnis zugunsten des letzteren verschoben ist, und unter vergleichbaren Bedingungen eine etwa um die Hälfte geringere Kohlendioxidabspaltung beobachtet wird.

Für das erfindungsgemäße Verfahren sind

ganz besonders geeignet zinnorganische Verbindungen, wie z.B.:

Trimethylzinnacetat, Triethylzinnbenzoat, Tributylzinnacetat, Triphenylzinnacetat, Dibutylzinndiacetat, Dibutylzinndilaurat, Dioctylzinndilaurat, Methoxytribytylzinn, Methoxytriphenylzinn, Phenoxytriethylzinn, Dimethoxydibutylzinn, Diethoxydibutylzinn, Diphenoxydibutylzinn, Dimethoxydiphenylzinn, Triethylzinnhydroxid, Triphenylzinnhydroxid, Hexaethylstannoxan, Hexabutylstannoxan, Tetrabutyldiphenoxystannoxan, Dibutylzinnoxid und Dioctylzinnoxid.

Vorzugsweise werden solche Verbindungen verwendet, deren Dampfdruck auch bei den erforderlichen Reaktionstemperaturen niedrig ist, d.h. solche mit metallorganisch gebundenen Alkylresten von mindestens vier Kohlenstoffatomen.

Als Dialkylcarbonate werden vorzugsweise solche der allgemeinen Formel (III)

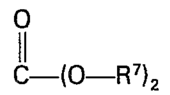

(III)

werwendet, in der $R^7$ für einen Alkylrest mit $C_1$—$C_{10}$ steht. Bevorzugt können Dimethylcarbonat, Diethylcarbonat, Dipropylcarbonat, Diisopropylcarbonat, Dibutylcarbonat, Dioctylcarbonat, Diisooctylcarbonat und Dicyclohexylcarbonat eingesetzt werden.

Als Phenole eignen sich vorzugsweise solche der allgemeinen Formel (IV)

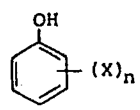

(IV)

in der X für Wasserstoff, einen Alkylrest mit $C_1$—$C_3$, ein Halogenatom, vorzugsweise Chlor, oder eine Nitrogruppe und n für 1 oder 2 stehen. Besonders bevorzugt werden als Phenole für das erfindungsgemäße Verfahren Phenol, o,m,p-Kresol, o,m,p-Chlorphenol, o,m,p-Ethylphenol, o,m,p-Propylphenol, o,m,p-Nitrophenol, 2,6-Dimethylphenol, 2,4-Dimethylphenol und 3,4-Dimethylphenol verwendet.

Anstelle der einwertigen Phenole können auch Bisphenole wie Dihydroxydiarylalkane mit $C_1$—$C_4$ im Alkylrest, so z.B. Bisphenol A, eingesetzt werden. Sie werden unter den Verfahrenbedingungen nicht gespalten und können so unmittelbar in Polycarbonat übergeführt werden. Diese sind, geht man von reinen Ausgangsstoffen aus, nahezu farblos und bedürfen keiner zusätzlichen Reinigungsschritte.

Die Katalysatoren werden in Konzentrationen von etwa 0,001—20 Gew.-%, bezogen auf die gesamte Menge des Reaktionsgutes, angewendet. Das Gewichtsverhältnis von Dialkylcarbonat: Phenol kann in weiten Grenzen schwanken und zwischen etwa 1:99 und 99:1, vorzugsweise 1:9 und 9:1 liegen. Von diesem Verhältnis hängt es ab, ob im Endprodukt Alkylphenylcarbonat oder Diarylcarbonat überwiegt.

Ohne Schwierigkeit kann das neben Diarylcarbonat gebildete Alkylarylcarbonat durch Destillation abgetrennt und entweder mit frischem Phenol umgesetzt oder nach Abtrennung des Diarylcarbonats mit dem noch vorhandenen Phenol weiterreagieren.

Die Reaktionstemperaturen liegen vorzugsweise im Bereich von 50—250°C, besonders bevorzugt im Bereich von 100—200°C. Vorteilhafterweise wird bei einem Druck von 1 Torr bis 20 Atm abs, vorzugsweise 1—5 Atm gearbeitet.

Lösungsmittel wie aliphatische oder aromatische Kohlenwasserstoffe können mitverwendet werden.

Eine bevorzugte Verfahrensweise besteht darin, das Umesterungsgemisch an einer längeren Kolonne auf die gewünschte Reaktionstemperatur zu bringen, während der Alkohol über Kopf in dem Maße, wie er im Reaktionsgut freigesetzt wird gegebenenfalls mit Hilfe eines inerten Gasstroms abgetrennt wird.

In einer anderen Verfahrensvariante wird überschüssiges Dialkylcarbonat durch eine Schmelze des umzusetzenden Phenols geleitet, während ein aus dem Alkohol und Dialkylcarbonat bestehendes Gemisch laufend abdestilliert. Die Trennung der Komponenten kann in einem separaten Schritt nach üblichen Methoden erfolgen.

Die Ergebnisse beider Verfahrensweisen unterscheiden sich nicht wesentlich.

Die Verfahrensprodukte können als Ausgangsstoffe zur Herstellung von Polycarbonaten nach bekannten Verfahren oder von Pflanzenschutzmitteln verwendet werden.

## Beispiel 1

a) In einer 2,9 m hohen, mit Glasringen beschickten verspiegelten Füllkörperkolonne werden 470 g (5 Mol) Phenol, 90 g (1 Mol) Dimethylcarbonat, 50 g n-Heptan und 5,7 g Diphenoxydibutylzinn zum Sieden erhitzt. Durch Eintropfen von Heptan wird die Innentemperatur bei 155°C gehalten. Ein Gemisch von Methanol und Heptan wird bei 59,5—60°C über Kopf destilliert. In dem Maße, wie die Reaktion voranschreitet, wird weiteres Dimethylcarbonat im unteren Kolonnendrittel eingetropft. Insgesamt werden so innerhalb von 30 h 315 g = 3,5 Mol Dimethylcarbonate eingesetzt. Etwa während der Reaktion abgespaltenes Kohlendioxid wird durch einen schwachen Stickstoffstrom in einer mit n-Natronlauge gefüllten Waschflasche absorbiert. Das Reaktionsgut wird über eine 1,1 m hohe Kolonne fraktioniert. Nach einem aus Methanol, Dimethylcarbonat und Heptan bestehenden Vorlauf gehen bei 80—87°C/15 Torr 313 g (3,33 Mol) unumgesetztes Phenol, bei 95—97°C/13 Torr 118 g (0,78 Mol) Methylphenylcarbonat ($n_D^{20}$ 1,4970) und nach Entfernen der Kolonne bei 165—172°C/13 Torr

90 g (0,44 Mol) kristallines Diphenylcarbonat über. Somit beträgt die Ausbeute, bezogen auf umgesetztes Phenol, 98,5% d. Th. In der Waschflasche sind nach der analytischen $CO_2$-Bestimmung 0,011 Mol Kohlendioxid absorbiert, das entspricht einem Verlust von 0,9% des eingesetzten Carbonats.

b) Ein Gemisch von 45,6 g (0,2 Mol) 2,2 - Bis - (4 - hydroxyphenyl) - propan, 47,1 g (0,22 Mol) des nach a) hergestellten Diphenylcarbonats und 0,008 g Natriummethylat wird langsam bis auf 210° unter 20 Torr erhitzt, wobei die Hauptmenge des abgespaltenen Phenols abdestilliert. Dann wird der Druck auf 0,2 Torr ermäßigt und die Temperatur während einer Stunde auf 250°C und während zweier weiterer Stunden auf 280°C erhöht, bis die Schmeize so zäh geworden ist, daß sie sich kaum mehr rühren läßt. Beim Abkühlen erhält man einen klaren, farblosen, elastischen Kunststoff, aus dessen Schmelze Formkörper mit hervorragenden Festigkeitseigenschaften hergestellt werden können.

Beispiel 2

An einer 2,3 m hohen verspiegelten Füllkörperkolonne werden 470 g (5 Mol) Phenol, 118 g (1 Mol) Diethylcarbonat, 200 g Xylol und 4 g Dimethoxydibutylzinn zum Sieden erhitzt. Über Kopf geht bei 78—80°C Ethanol über. In den unteren Kolonnenteil wird so viel Diethylcarbonat eingetropft, daß die Innentemperatur bei 157—158°C gehalten wird. Insgesamt werden im Laufe von 28 h 2,5 Mol Diethylcarbonat eingebracht, während 1,8 Mol Ethanol abdestilliert werden. Ein schwacher Stickstoffstrom führt das Abgas durch eine mit n-Natronlauge beschickte Waschflasche. Das Reaktionsgut wird über eine 1 m Kolonne fraktioniert. Nach Abdestillieren von Xylol und nichtumgesetztem Diethylcarbonat gehen bei 76—80°C/13 Torr 302 g Phenol über. Der Rückstand weist eine schwach graue Färbung auf. Bei 102—107°C/12 Torr destillieren Ethylphenylcarbonat (113 g = 0,68 Mol; $n_D^{20}$ 1,4871) und nach Entfernung der Kolonne bei 165—170°C/13 Torr 110 g (0,54 Mol) Diphenylcarbonat. Die Ausbeute, bezogen auf umgesetztes Phenol, beträgt über 99% d. Th. Während der Reaktion werden 0,005 Mol $CO_2$ abgespalten, das entspricht einem Verlust von 0,41% Carbonat.

Beispiel 3 (Vergleichsbeispiel)

In der gleichen Weise, wie in Beispiel 2 beschrieben, werden 5 Mol Phenol mit 2,5 Mol Diethylcarbonat unter Verwendung von 4 g Titanetrabutylat als Katalysator innerhalb 28 h bei einer Innentemperatur von 157—158°C umgesetzt.

298 g Phenol werden zurückgewonnen. 149 g (0,9 Mol) Ethylphenylcarbonat und 92 g (0,43 Mol) Diphenylcarbonat werden erhalten.

Die Ausbeute, bezogen auf umgesetztes Phenol, beträgt somit 96% d. Th. Während der Reaktion werden 0,014 Mol $CO_2$ abgespalten. Das entspricht einem Verlust an Carbonat von 1,05%.

Das Reaktionsprodukt ist vor der Destillation tief rot-braun gefärbt. Auch nach der Destillation weist das Diphenylcarbonat noch einen rot-braunen Stich auf.

Beispiel 4

In einer Apparatur wie im Beispiel 2 beschrieben werden 1880 g (20 Mol) Phenol, 300 g (2,54 Mol) Diethylcarbonat und 20 g Tetrabutyldiphenoxystannoxan unter Überleiten von Stickstoff zum Sieden erhitzt, bis über Kopf bei 78—79,5°C Ethanol abdestilliert. Im unteren Kolonnenteil wird Diethylcarbonat derart zugesetzt, daß die Sumpftemperatur bei 176°C—178°C gehalten wird. Im Laufe von 25 h kommen insgesamt 644 g (8 Mol) Diethylcarbonat zur Anwendung. 253 g (5,5 Mol) Ethanol werden abdestilliert. Nach Abdestillieren des nicht umgesetzten Diethylcarbonats gehen bei 82—88°C/20 Torr 1339 g Phenol, bei 114—118°C/20 Torr 291 g (1,75 Mol) Ethylphenylcarbonat und 391 g (1,825 Mol) Diphenylcarbonat über. Die Ausbeute, bezogen auf umgesetztes Phenol, beträgt somit 98,5% d. Th. Im Abgas werden 1,35 g (0,035 Mol) Kohlendioxid nachgewiesen, das entspricht einem Verlust von 1% Carbonat.

**Patentansprüche**

1. Verfahren zur Herstellung von aromatischen Kohlensäureestern durch Umesterung von Dialkylcarbonaten mit Phenolen unter Abspaltung von Alkoholen in Gegenwart von Umesterungskatalysatoren, dadurch gekennzeichnet, daß man als Umesterungskalalysatoren Organozinnverbindungen der Allgemeinen Formel (I)

$$(R^1)_{4-x}—Sn—(Y)_x \qquad (I),$$

in der

Y für einen Rest

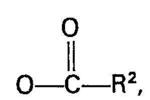

OH oder $OR^2$ steht, wobei $R^2$ einen Alkylrest mit $C_1$—$C_{12}$, einen Arylrest mit $C_6$—$C_{12}$ oder einen Alkylarylrest mit $C_7$—$C_{13}$ bedeutet, und

$R^1$ die Bedeutung von $R^2$ hat, und

x eine ganze Zahl von 1—3 bedeutet,

oder Dialkylzinnoxide mit jeweils 1—12 C-Atomen im Alkylrest oder zinnorganische Verbindungen der allgemeinen Formel (II)

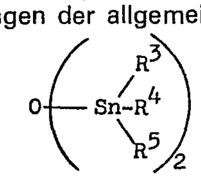

(II),

in der

$R^3$ und $R^4$ gleich oder verschieden, die oben angegebene Bedeutung von $R^2$ haben, und

$R^5$ die Bedeutung von $R^2$ hat oder für einen Rest $OR^6$ steht, in dem $R^6$ die Bedeutung von $R^2$ hat,

in Mengen von 0,001 bis 20 Gew.-%, bezogen auf die Gesamtmenge des Reaktionsgutes, verwendet.

2. Verfahren nach Anspruch 1, dadurch gekennzeichnet, daß als Organozinnverbindungen Trimethylzinnacetat, Triethylzinnbenzoat, Tributylzinnacetat, Triphenylzinnacetat, Dibutylzinndiacetat, Dibutylzinndilaureat, Dioctyl zinndilaureat, Methoxytributylzinn, Methoxytriphenylzinn, Phenoxytriethylzinn, Dimethoxydibutylzinn, Diethoxydibutylzinn, Diphenoxydibutylzinn, Dimethoxydiphenylzinn, Triethylzinnhydroxid, Triphenylzinnhydroxid, Hexaethylstannoxan, Hexabutylstannoxan, Tetrabutyldiphenoxystannoxan, Dibutylzinnoxid oder Dioctylzinnoxid eingesetzt werden.

3. Verfahren nach Ansprüchen 1 und 2, dadurch gekennzeichnet, daß die Umesterung bei Temperaturen von 50 bis 250°C erfolgt.

**Claims**

1. A process for the production of aromatic carbonic acid esters by transesterifying dialkyl carbonates with phenols under the elimination of alcohols and in the presence of transesterification catalysts, wherein organo tin compounds of the general formula I

$$(R^1)_{4-x}—Sn—(Y)_x \quad (I),$$

in which

Y represents an

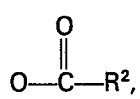

OH or $OR^2$ radical, $R^2$ denoting a $C_1—C_{12}$ alkyl radical, a $C_6—C_{12}$ aryl radical or a $C_7—C_{13}$ alkylaryl radical, and

$R^1$ has the meaning of $R^2$ and

x denotes an integer from 1 to 3,

or dialkyl tin oxides each with 1—12 C-Atoms in the alkyl radical or organo tin compounds of the general formula II

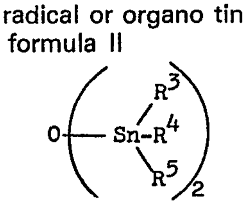

(II),

in which

$R^1$ and $R^4$ are the same or different and have the above-indicated meaning of $R^2$, and

$R^5$ has the meaning of $R^2$ or represents a radical $OR^6$ in which $R^6$ has the meaning of $R^2$

are used as reesterification catalysts, in quantities of from 0.001 to 20% by weight, based on the total amount of the reaction mixture.

2. A process according to claim 1, wherein as organo tin compounds trimethyl tin acetate, triethyl tin benzoate, tributyl tin acetate, triphenyl tin acetate, dibutyl tin diacetate, dibutyl tin dilaurate, dioctyl tin dilaurate, methoxy tributyl tin, methoxy triphenyl tin, phenoxy triethyl tin, dimethoxy dibutyl tin, diethoxy dibutyl tin, diphenoxy dibutyl tin, dimethoxy diphenyl tin, triethyl tin hydroxide, hexa-ethyl stannoxane, tetrabutyl diphenoxy stannoxane, dibutyl tin oxide or dioctyl tin oxide are used.

3. A process according to claims 1 and 2, wherein the reesterification takes place at temperatures of from 50 to 250°C.

**Revendications**

1. Procédé pour la préparation d'esters carboniques aromatiques par transestérification de carbonates de dialkyle avec des phénols avec libération d'alcools en présence de catalyseurs, caractérisé en ce qu'on utilise comme catalyseurs des composés organiques d'étain de formule générale

$$(R^1)_{4-x}—Sn—(Y)_x \quad (I),$$

dans laquelle

Y représente un reste

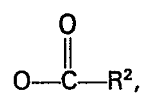

OH ou $OR^2$, où $R^2$ représente un reste alkyle en $C_1 — C_{12}$, un reste aryle en $C_6 — C_{12}$ ou un reste alkylaryle en $C_7—C_{13}$, et

$R^1$ a la signification de $R^2$, et

x est un nombre entier de 1 à 3,

ou des oxydes de di(alkyle en $C_1—C_{12}$) étain ou des composés organiques d'étain de formule générale

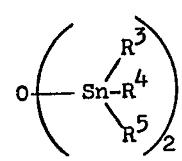

(II)

dans laquelle

$R^3$ et $R^4$ sont identiques ou différents et ont la signification de $R^2$ indiqués ci-dessus, et

$R^5$ a la signification de $R^2$ ou représente un reste $OR^6$ dans lequel $R^6$ a la signification de $R^2$,

en quantités de 0,001 à 20% en poids par rapport à la quantité totale du mélange de réaction.

2. Procédé selon la revendication 1, caractérisé en ce qu'on utilise comme composés d'organoétain: acétate de triméthylétain, benzoate de triéthylétain, acétate de tributylétain, diacétate de triphénylétain, dilaurate de dibutylétain, dilaurate de dibutylétain, dioctyl-

étain, méthoxytributylétain, méthoxytriphénylétain, phénoxytriéthylétain, diméthoxydibutylétain, diéthoxydibutylétain, diphénoxydibutylétain, diméthoxydiphénylétain, hydroxyde de triéthylétain, hydroxyde de triphénylétain, hexaéthylstannoxanne, hexabutylstannoxanne, tétrabutyldiphénoxystannoxanne, oxyde de dibutylétain et de dioctylétain.

3. Procédé selon les revendications 1 et 2, caractérisé en ce que l'on effectue la transestérification à des températures de 50 à 250°C.